# EUROPEAN PATENT APPLICATION

(11) **EP 2 343 076 A1**
(43) Date of publication of application: **13.07.2011**
(21) Application number: 09817722.3
(22) Date of filing: 28.09.2009
(51) Int. Cl.: A61K 31/551, A61K 9/20, A61K 47/38, A61P 7/02, A61P 9/10

(54) **PHARMACEUTICAL COMPOSITION FOR ORAL ADMINISTRATION**

(30) Priority: 30.09.2008 US 101356 P
(71) Applicant: Astellas Pharma Inc., Tokyo 103-8411 (JP)
(72) Inventor: OBA Shinsuke, Tokyo 103-8411 (JP); YASUJI Takehiko, Tokyo 103-8411 (JP); HAKOMORI Tadashi, Tokyo 103-8411 (JP); SAKO Kazuhiro, Tokyo 103-8411 (JP)
(74) Representative: HOFFMANN EITLE
(86) International application number: PCT/JP2009/066741
(87) International publication number: WO 2010/038689

(57) **Abstract**

A pharmaceutical composition for oral administration, comprising 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof, and hydroxypropyl cellulose having a viscosity of approximately 6 mPa•S (inclusive) to approximately 150 mPa•S (exclusive) in a 2% aqueous solution at 20°C, or a viscosity of 75 mPa•S (inclusive) to 400 mPa•S (inclusive) in a 5% aqueous solution at 25°C, and a process of manufacturing the pharmaceutical composition for oral administration are disclosed.

## Description

### TECHNICAL FIELD

The present invention relates to a pharmaceutical composition for oral administration containing a diazepan derivative. More particularly, the present invention relates to a pharmaceutical composition for oral administration containing 3-hydroxy-N¹-(4-methoxybenzoyl)-N-[4-(4-methyl-1,4-diazepan-1-yl)benzoyl]-1,2-phenylenediamine or a pharmaceutically acceptable salt thereof, wherein disintegrability is maintained and failures in tabletting (such as sticking to punches under compression-molding) are reduced by adding specific hydroxypropyl cellulose as a binder to the pharmaceutical composition, and a process of manufacturing the same.
Further, the present invention relates to a use of specific hydroxypropyl cellulose in the manufacture of a pharmaceutical composition for oral administration, wherein disintegrability is maintained and sticking to punches is prevented by adding the specific hydroxypropyl cellulose as a binder to 3-hydroxy-N¹-(4-rnethoxybenzoyl)-N²- [4-(-4-methyl-1,4-diazepan-1-yl)benzoyl]-1,2-phenylenediamine or a pharmaceutically acceptable salt thereof.

### BACKGROUND ART

Diazepan derivatives, 3-hydroxy-N¹-(4-methoxybenzoyl)-N²-[4-(4-methyl-1,4-diazepan-1-yl)benzoyl]-1,2-phenylenediamine or pharmaceutically acceptable salts thereof, are compounds which were newly generated by Astellas Pharma Inc., and specifically inhibit activated blood coagulation factor Xa and have a potent anticoagulant effect . Development of the compounds as a blood anticoagulant, or a medicament for the treatment or prevention of diseases caused by a thrombus or embolus is being examined (patent literature 1).

Pharmaceutical formulations are produced in multiple steps consisting of unit procedures such as pulverization, mixing, granulation, drying, tabletting, surface modification, and the like, and are provided to the medical field. When a medicament is an ordinary formulation, a good disintegrability and a good solubility are needed. When these properties are imparted to a pharmaceutical formulation, failures in tabletting (sticking and binding) are caused in the production steps, particularly during the tabletting step, and in some cases leads to a failure in molding, such as the occurrence of chipped tablets. In an industrial scale production, sticking and binding become a cause for a breakdown of a machine and it is forecast that the production per se will become difficult. Further, when an identification code is to be impressed on the surface of a tablet, the sticking and binding cause a missing impression, which is a serious problem. Furthermore, when such a sticking to punches occurs in the commercial scale production, it is necessary to stop the production and remove the stuck material from the surface of the punch. If such a trouble frequently occurs in the commercial scale production, various problems such as a reduced production efficiency, or a reduction in quality caused by increased defective tablets (a reduction in yield) are caused.

The sticking and binding are generally prevented by increasing the amount of a lubricant such as talc, magnesium stearate, or the like. However, an increased amount of a lubricant reduces the moldability of a formulation per se and, in some cases, causes a reduction in hardness or a decrease in friability. Thus, it is desired that the problem is solved by avoiding this method, if possible. The sticking to punches and dies can be prevented by polishing the surfaces of a punch and a die used in the tabletting process, but the effect of polishing is not permanent, and thus, the sticking and binding occurs by an abrasion, and the problem cannot be fundamentally solved by this method.

In order to alleviate sticking to punches, a pharmaceutical formulation prepared by mixing a punch-sticking drug bulk with granules coated with a coating composition containing a plasticizer, and forming the mixture into tablets, is disclosed (patent literature 2).
In order to provide a composition for tablet capable of reversibly preventing the sticking of a physiologically active component having a melting point of 70 to 150°C and capable of forming a mixture containing the component into tablets, and a tabletting method, a composition for tablet characterized by formulating crystalline powder having an average particle size of to 100 µm is disclosed (patent literature 3).

However, the techniques described in these literatures are techniques which prevent sticking to punches in the production of a pharmaceutical formulation, but are not techniques which maintain a good disintegrability as a pharmaceutical formulation and alleviate sticking to punches under compression-molding. With respect to a technique and a pharmaceutical composition of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof to maintain the disintegrability and alleviate sticking to a (metal) punch, and a process of manufacturing the same, further development is desired.

### CITATION LIST

### PATENT LITERATURE

[patent literature 1] International Publication No. WO 2001/074791 (Japanese Patent No. 3788349)
[patent literature 2] Japanese Unexamined Patent Publication (Kokai) No. 2007-169273
[patent literature 3] Japanese Unexamined Patent Publication (Kokai) No. 10-59842

### SUMMARY OF INVENTION

### TECHNICAL PROBLEM

During the formulation study, the present inventors found that 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof exhibited a high punch-sticking property.
An object of the present invention is to provide a pharmaceutical composition for oral administration in which disintegrability of a compound having a high punch-sticking property, 3-hydroxy-N¹-(4-methoxybenzoyl)-N-[4-(4-methyl 1,4-diazepan-1-yl)benzoyl]-1,2-phenylenediamine or a pharmaceutically acceptable salt thereof, is maintained and failures in tabletting (such as sticking to punches) are prevented, and a process of manufacturing the pharmaceutical composition.
Another object of the present invention is to provide a use of specific hydroxypropyl cellulose in the manufacture of a pharmaceutical composition for oral administration, wherein disintegrability is maintained and failures in tabletting (such as sticking to punches) are prevented by adding the hydroxypropyl cellulose as a binder to 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof.

### SOLUTION TO PROBLEM

Under these circumstances, the present inventors conducted intensive studies on a pharmaceutical composition containing 3-hydroxy-N¹-(4-methoxybenzoyl)-N2-[4-(4-methyl-1,4-diazepan-1-yl)benzoyl]-1,2-phenylenediamine or a pharmaceutically acceptable salt thereof, and found that conventional formulations resulted in failures in tabletting (sticking and binding) under compression-molding, such as sticking to a punch or a die. Further, the present inventors found that some components added to the pharmaceutical composition ameliorated the failure in tabletting such as sticking to punches, but decreased the disintegrability of the tablet, and as a result, a delay in dissolution of the drug was caused. The present inventors conducted intensive studies to solve these problems, and found that the failure in tabletting under compression-molding, such as sticking to punches, could be reduced and a good disintegrability of the tablet could be maintained by selecting and using a specific water-soluble polymer, to complete the present invention.

The present invention provides:
[1] a pharmaceutical composition for oral administration, comprising 3-hydroxy-N¹-(4-rnethoxybenzoyl)-N²-[4-(4-methyl-1,4-diazepan-1-yl)benzoyl]-1,2-phenylenediamine or a pharmaceutically acceptable salt thereof, and hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C,
[2] the pharmaceutical composition for oral administration according to [1], wherein the viscosity of hydroxypropyl cellulose is approximately 6 mPa·S (inclusive) to approximately 10 mPa·S (inclusive) in a 2% aqueous solution at 20°C,
[3] the pharmaceutical composition for oral administration according to [1] or [2], wherein the amount of hydroxypropyl cellulose is approximately 2 w/w% to approximately 720 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof,
[4] the pharmaceutical composition for oral administration according to any one of [1] to [3], wherein the amount of hydroxypropyl cellulose is approximately 4 w/w% to approximately 360 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof,
[5] the pharmaceutical composition for oral administration according to any one of [1] to [4], wherein the amount of hydroxypropyl cellulose is approximately 7 w/w% to approximately 25 w/w% with respect to the amount of 3-hydroxy-N¹-(4-methaxybenzoyl) -N²-[4-(4-methyl-1,4-diazepan-1-yl)benzoyl]-1,2-phenylenediamine or a pharmaceutically acceptable salt thereof,
[6] the pharmaceutical composition for oral administration according to any one of [1] to [5], characterized in that the composition is disintegrated within 5 minutes,
[7] a process of manufacturing a pharmaceutical composition for oral administration, comprising mixing 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof with hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C,
[8] the process according to [7], wherein the viscosity of hydroxypropyl cellulose is approximately 6 mPa·S (inclusive) to approximately 10 mPa·S (inclusive) in a 2% aqueous solution at 20°C,
[9] the process according to [7] or [8], wherein the amount of hydroxypropyl cellulose is approximately 2 w/w% to approximately 720 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof,
[10] the process according to any one of [7] to [9], wherein the amount of hydroxypropyl cellulose is approximately 4 w/w% to approximately 360 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof,
[11] the process according to any one of [7] to [10], wherein the amount of hydroxypropyl cellulose is approximately 7 w/w% to approximately 25 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof,
[12] the process according to any one of [7] to [11], characterized in that the composition is disintegrated within 5 minutes, and
[13] use of hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C in the manufacture of a pharmaceutical composition for oral administration, wherein disintegrability is maintained and sticking to punches under compression-molding is reduced by adding the hydroxypropyl cellulose as a binder to 3-hydroxy-N'-(4_ methoxybenzoyl)-N²-[4-(4-methyl-1,4-diazepan-1-yl)benzoyl]-1,2-phenylenediamine or a pharmaceutically acceptable salt thereof.

### ADVANTAGEOUS EFFECTS OF INVENTION

According to the present invention, a pharmaceutical formulation capable of (1) maintaining a good disintegrability of the drug, and (2) reducing failures in tabletting such as sticking to punches under compression-molding can be provided.

### DESCRIPTION OF EMBODIMENTS

Hereinafter the pharmaceutical composition for oral administration will be explained.
The term "sticking to punches" as used herein includes the phenomenon that a drug or powder sticks to the surface of a punch in tabletting, and phenomena observed when the tabletting is further continued, for example, the phenomenon that a missing impression on the surface of a tablet is caused by material which sticks to a punch and a die, and the phenomenon that the surface of an uncoated tablet becomes rough or projections and/or depressions are formed on the surface of an uncoated tablet by the growth of attachment.
The evaluation of "sticking to punches" as used herein is carried out by performing tabletting using a tabletting machine, visually observing the surface of the punch, or observing the surface using a microscope in another embodiment, and judging in accordance with the presence or absence of blurring of the punch.

The evaluation of disintegrability as used herein is carried out in accordance with the general tests described in the Japanese Pharmacopoeia, using 6 tablets having a hardness of 50 N or more. More particularly, water is used as the test fluid. A test basket, in which a tablet is placed, is attached to a vertical axis, and arranged in a beaker so that the test basket can be smoothly raised and lowered through a distance between 53 mm and 57 mm at a constant frequency between 29 and 32 cycles per minute. The test basket is adjusted so that a wire mesh as the bottom face of the test basket is located 25 mm from the bottom of the beaker when the test basket is at the lowest point of the downward stroke. The volume of the test fluid in the beaker is such that the upper face of the test basket accords with the surface of the test fluid when the test basket is at the lowest point of the downward stroke. The temperature of the test fluid is maintained at 37±2°C, and the time necessary for the disintegration of each tablet is measured. The average of values measured for 6 tablets is regarded as the disintegration time.
Witch respect to the term "good disintegrability" as used herein, the disintegration time determined by the general tests is, for example, within 5 minutes, and within 4 minutes in another embodiment.

A drug which may be used in the present invention, 3-hydroxy-N¹-(4-methoxybenzoyl)-N²- [4-(4-methyl-1,4-diazepan-1-yl)benzoyl]-1,2-phenylenediamine (hereinafter sometimes abbreviated to compound A), is represented by the following structural formula, and diazepan derivatives including compound A are disclosed in Japanese Patent No. 3788349.

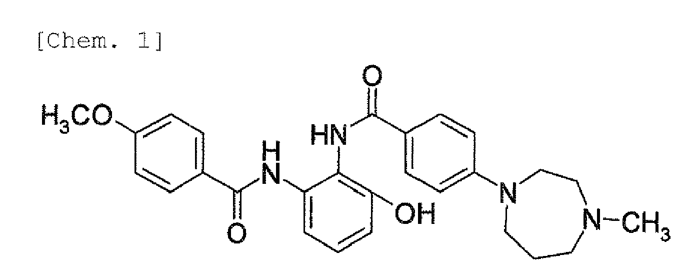

Compound A may be used in a free form which is not a salt, may form an acid addition salt or a salt with a base in another embodiment, and may form an acid addition salt in still another embodiment. More particularly, examples of such a salt include an acid addition salt with a mineral acid such as hydrochloric acid, hydrobromic acid, hydroiodic acid, sulfuric acid, nitric acid, phosphoric acid, or the like; an acid addition salt with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, or the like; an acid addition salt with an acidic amino acid such as aspartic acid, glutamic acid, or the like; a salt with an inorganic base such as sodium, potassium, magnesium, calcium, aluminum, or the like; a salt with an organic base such as methylamine, ethylamine, ethanolamine, or the like; a salt with a basic amino acid such as lysine, ornithine, or the like; and an ammonium salt. In another embodiment, a salt with maleic acid may be used.

The drug used in the present invention specifically inhibits activated blood coagulation factor X and has a potent anticoagulant effect. Therefore, the drug is useful as a blood anticoagulant, or a medicament for the treatment or prevention of diseases caused by a thrombus or embolus.

The clinical dose of compound A for a human is appropriately selected in accordance with symptoms, body weight, age, sex, and the like of the patient to be treated. In general, compound A is orally administered to an adult at a daily dose of, for example, 0.1 mg to 500 mg, 0.3 mg to 200 mg in another embodiment, and 1 mg to 120 mg in still another embodiment, which is administered once or divided into multiple doses per day. Since the dose varies under various conditions, a smaller dose than the above range may be sufficient in some cases.
The content of compound A is not particularly limited, so long as it is contained in an efficient amount per dosage unit formulation. For example, the content of compound A per formulation is, for example, 0.1% by weight to 55% by weight, and 0.5% by weight to 45% by weight in another embodiment.

Hydroxypropyl cellulose (hereinafter sometimes referred to as HPC) is a hydroxy ether obtainable by reacting cellulose with propylene oxide, and has a different viscosity depending on the degree of polymerization.
HPC used in the present invention is not particularly limited, so long as it is pharmaceutically acceptable, can reduce failures in tabletting such as sticking to punches, and can maintain a good disintegrability of the tablet. HPC has, for example, a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C; and a viscosity of approximately 6 mPa·S (inclusive) to approximately 10 mPa·S (inclusive) in a 2% aqueous solution at 20°C, or a viscosity of 75 mPa·S (inclusive) to 400 mPa·S (inclusive) in a 5% aqueous solution at 25°C in another embodiment.
As such a polymer, for example, HPC-L (product name; Nippon Soda Co., Ltd.) having a viscosity of approximately 6 mPa·S (inclusive) to approximately 10 mPa·S (inclusive) in a 2% aqueous solution at 20°C, KLUCEL LF (product name; Hercules Inc.) having a viscosity of 75 mPa·S (inclusive) to 150 mPa·S (inclusive) in a 5% aqueous solution at 25°C, and KLUCEL JF (product name; Hercules Inc.) having a viscosity of 150 mPa·S (inclusive) to 400 mPa·S (inclusive) in a 5% aqueous solution at 25°C, are commercially available.

In the present invention, HPC may be added to the pharmaceutical composition as a solution or a suspension prepared by dissolving or suspending HPC in a solvent such as water, or by physically mixing HPC with the drug.
The content of HPC is not particularly limited, so long as it is pharmaceutically acceptable, can reduce failures in tabletting such as sticking to punches, and can maintain a good disintegrability of the tablet. The content of HPC with respect to the weight of the drug is, for example, approximately 2 w/w% to approximately 720 w/w%, approximately 4 w/w% to approximately 360 w/w% in another embodiment, and approximately 7 w/w% to approximately 25 w/w% in still another embodiment. The content of HPC with respect to the total weight of the formulation is, for example, approximately 2 w/w% to approximately 10 w/w%, approximately 3 w/w% to approximately 7.5 w/w% in another embodiment, and approximately 3 w/w% to approximately 5 w/w% in still another embodiment.

The pharmaceutical composition for oral administration of the present invention is formulated appropriately using various further pharmaceutical additives, if desired. Such pharmaceutical additives are not particularly limited, so long as they are pharmaceutically acceptable, and include, for example, fillers, binders, disintegrating agents, acidulants, foaming agents, artificial sweeteners, flavors, lubricants, coloring agents, stabilizers, buffers, antioxidants, surfactants, and the like.

Examples of the binders include hydroxypropyl methylcellulose, hydroxypropyl cellulose, polyvinyl alcohol, methylcellulose, gum arabic, and the like.
Examples of the fillers include lactose, starch, corn starch, microcrystalline cellulose, mannitol, light anhydrous silicic acid, magnesium carbonate, calcium carbonate, sucrose, glucose, and the like.
Examples of the disintegrating agents include croscarmellose sodium, low substituted hydroxypropylcellulose, carmellose, methylcellulose, carboxymethyl starch sodium, crospovidone, partly pregelatinized starch, corn starch, potato starch, carmellose calcium, carmellose sodium, and the like. In another embodiment, examples of the disintegrating agents include croscarmellose sodium, low substituted hydroxypropylcellulose, carboxymethyl starch sodium, and crospovidone.
Examples of the acidulants include citric acid, tartaric acid, malic acid, and the like.
Examples of the foaming agents include sodium bicarbonate and the like.
Examples of the artificial sweeteners include saccharin sodium, dipotassium glycyrrhizinate, aspartame, stevia, somatin, and the like.
Examples of the flavors include lemon, lemon lime, orange, menthol, and the like.
Examples of the lubricants include magnesium stearate, calcium stearate, sucrose fatty acid ester, polyethylene glycol, talc, stearic acid, and the like.
Examples of the coloring agents include yellow ferric oxide, red ferric oxide, food yellow No. 4, food yellow No. 5, food red No. 3, food red No. 102, food blue No. 3, and the like.
Examples of the buffers include citric acid, succinic acid, fumaric acid, tartaric acid, ascorbic acid, and salts thereof; glutamic acid, glutamine, glycine, aspartic acid, alanine, arginine, and salts thereof; magnesium oxide, zinc oxide, magnesium hydroxide, phosphoric acid, boric acid, and salts thereof; and the like.
Examples of the antioxidants include ascorbic acid, dibutyl hydroxytoluene, propyl gallate, and the like.
Examples of the surfactants include polysorbate 80, sodium lauryl sulfate, polyoxyethylene hydrogenated castor oil, and the like.

These pharmaceutical additives may be appropriately added alone, or as a combination of two or more thereof, in appropriate amounts. The content of these additives is 1% by weight (inclusive) to 100% by weight (exclusive) with respect to the total weight of the formulation, 20% by weight (inclusive) to 100% by weight (exclusive) in another embodiment, and 40% by weight (inclusive) to 100% by weight (exclusive) in still another embodiment. In this regard, with respect to the binders, an embodiment in which an amount of the specific HPC used in the present invention is added so that the desired effects caused by the specific HPC are obtained is not excluded.

The pharmaceutical composition of the present invention may be used to prepare various pharmaceutical formulations, which include, for example, powder, granules, dry syrups, capsules, tablets, rapidly disintegrating tablets in the buccal cavity, and the like.
Fine particles used for preparing powder, granules, dry syrups, capsules, or the like are useful, because they have excellent flowability, and the dose can be controlled.

Hereinafter a process of manufacturing the pharmaceutical composition of the present invention will be described, but the present invention is not limited thereto.
The pharmaceutical composition of the present invention can be produced in accordance with a known method per se, such as pulverization, mixing, granulation, tabletting, film coating, or the like.
The method of pulverization is not particularly limited with respect to apparatus and procedures, so long as it is a conventional method in which pulverization can be pharmaceutically carried out. Examples of a pulverizer include a hammer mill, a ball mill, a jet mill, and the like. The conditions for pulverization may be appropriately selected and are not particularly limited.

The method of mixing is not particularly limited with respect to apparatus and procedures, so long as it is a conventional method in which each component can be pharmaceutically and uniformly mixed. Examples of a mixer include a V type mixer, a ribbon type mixer, a container mixer, a high speed mixer, and the like. The conditions for mixing may be appropriately selected and are not particularly limited.

As the method of granulating the drug, a conventional granulation method may be used. Examples of such a conventional granulation method include a fluidized bed granulation method, an agitation granulation method, a high-shear granulation method, a tumbling fluidized bed granulation method, an extrusion granulation method, a pulverization granulation method, a dry granulation method, and the like. In another embodiment, examples thereof include a fluidized bed granulation method, an agitation granulation method, a high-shear granulation method, and a tumbling fluidized bed granulation method, and any method capable of granulating the drug may be used.

For example, in accordance with a fluidized bed granulation method, while the drug is fluidized together with one or more additives such as a filler or the like, an appropriate amount of liquid containing hydroxypropyl cellulose may be sprayed using a spray gun. The liquid containing hydroxypropyl cellulose is prepared by dissolving or dispersing hydroxypropyl cellulose in a solvent such as water, ethanol, methanol, or the like. These solvents may be used as an appropriate mixture.

The concentration of the HPC solution is 1% to 20% as a solid content, and 5% to 15% in another embodiment.

A preferred spray rate of the binder liquid is not particularly limited, so long as a nonuniform mixture consisting of untreated powder and aggregates, which are generally powdery, is not generated. The spray rate varies in accordance with a production method or a scale for production, but is 1 g/min to 20 g/min, 5 g/min to 20 g/min in another embodiment, and 8 g/min to 12 g/min in still another embodiment for a 1 kg-scale production using a fluidized bed granulation method.

A preferred temperature of the product in granulation is 20°C to 40°C, and 25°C to 35°C in another embodiment.
The granulated product may be further dried, heated, or the like, and a preferred temperature of the granulated product in this treatment is, for example, 30°C to 50°C, and 40°C to 45°C in another embodiment.

The method of tabletting is not particularly limited, so long as it is a conventional method for pharmaceutically producing a compression-molded product. Examples of the method include a direct tabletting method in which the drug and hydroxypropyl cellulose are mixed with an appropriate additive(s), and the mixture is compression-molded to obtain tablets; a method in which a product obtained by granulation is mixed with a lubricant or the like, and the mixture is formed into tablets; and the like.

The apparatus for tabletting is not particularly limited, so long as a compression-molded product (preferably a tablet) can be pharmaceutically produced. Examples of the apparatus include a rotary tabletting machine, a single punch tabletting machine, and the like.

After the tabletting, the surface of each tablet may be coated with a film.
The method of film-coating is not particularly limited, so long as tablets can be pharmaceutically coated. Examples of the method include pan coating, dip coasting, and the like.

The film-coating agent is not particularly limited, so long as it is a pharmaceutical additive for coating. Examples of the film-coating agent include hydroxypropyl methylcellulose, hydroxypropyl cellulose, hydroxypropylmethyl cellulose phthalate, methacrylate copolymer L, methacrylate copolymer LD, methacrylate copolymer S, methyl cellulose, ethyl cellulose, stearic acid, stearyl alcohol, macrogol, propylene glycol, triacetin , glycerin, titanium dioxide, talc, carnauba wax, and the like. These film-coating agents may be appropriately added alone, or as a combination of two or more thereof, in appropriate amounts.
The coating rate is not particularly limited, so long as a normal coating rate is used. The coating rate is, for example, 1% by weight to 5% by weight with respect to the weight of an uncoated tablet, and 2% by weight to 4% by weight in another embodiment.

The use of hydroxypropyl cellulose according to the present invention is a use of hydzaxypropyl cellulose having a viscosity of approximately 6 mPa·S (inclusive) to approximately 150 mPa·S (exclusive), or a viscosity of 75 mPa·S (inclusive) to 400 mPa·S (inclusive) in a 5% aqueous solution at 25°C in the manufacture of a pharmaceutical composition for oral administration, wherein disintegrability is maintained and sticking to punches under compression-molding is reduced by adding the hydroxypropyl cellulose as a binder to compound A or a pharmaceutically acceptable salt thereof. The description of the pharmaceutical composition of the present invention is cited as embodiments to carry out the use of the present invention.

### EXAMPLES

The present invention will be further illustrated by, but is by no means limited to, the following Examples and comparative Examples. Compound A maleate, the salt of compound A with maleic acid, was prepared in accordance with the method described in International Publication WO 2001/074791 (Japanese Patent No. 3788349) for use in the following Examples.

### Example 1

A binder liquid was prepared by dissolving 2.7 parts of hydroxypropyl cellulose having a viscosity of approximately 6 mPa·S (inclusive) to approximately 10 mPa·S (inclusive) in a 2% aqueous solution at 20°C (manufactured by Nippon Soda Co., Ltd.; product name: HPC-L; The same compound was used in the following Examples, unless otherwise specified.) in 31.05 parts of purified water. Into a fluidized bed granulating apparatus (manufactured by Powrex; GPCG-5/15; The same apparatus was used in the following Examples.), 12.45 parts of pulverized compound A maleate and 62.25 parts of D-mannitol (manufactured by Roquette; product name: Pearlitol 50C; The same compound was used in the following Examples.) were loaded, and sprayed with the binder liquid at a spray rate of 100 g/min to obtain granules. After the binder spraying, the resulting granules were dried to obtain a pharmaceutical composition (granulated product) of the present invention. Into a vessel-rotary mixer (manufactured by Kotobuki Industries Co., Ltd.; Container mixer LM-20; The same apparatus was used in the following Examples.), 77.4 parts of the pharmaceutical composition (granulated product) of the present invention, 9 parts of microcrystalline cellulose (manufactured by Asahi Kasei Chemicals Corporation; product name: Ceolus PH-102; The same compound was used in the following Examples.), 2.7 parts of croscarmellose sodium (manufactured by FMC; product name: Ac-Di-Sol; The same compound was used in the following Examples.), and 0.9 parts of magnesium stearate (manufactured by Merck; The same compound was used in the following Examples.) were loaded, and mixed. A rotary tabletting machine (manufactured by Hata Iron Works Co., Ltd.; HT-X-SS-20; The same apparatus was used in the following Examples.) was used to obtain a pharmaceutical composition (tablets) of the present invention (a punch of 6 mm in diameter; a compression pressure of 6 kN/punch; a tablet weight of 90 mg).

### Example 2

A binder liquid was prepared by dissolving 2.7 parts of hydroxypropyl cellulose in 31.05 parts of purified water. Next, 37.35 parts of pulverized compound A maleate and 37.35 parts of D-mannitol were loaded into a fluidized bed granulating apparatus, and sprayed with the binder liquid at a spray rate of 100 g/min to obtain granules at a product temperature of 28°C. After the binder spraying, the resulting granules were dried to obtain a pharmaceutical composition (granulated product) of the present invention. Into a vessel-rotary mixer, 77.4 parts of the pharmaceutical composition (granulated product) of the present invention, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) of the present invention (a punch of 6 mm in diameter; a compression pressure of 6 kN/punch; a tablet weight of 90 mg).

### Example 3

A binder liquid was prepared by dissolving 5.4 parts of hydroxypropyl cellulose in 62.1 parts of purified water. Next, 74.7 parts of pulverized compound A maleate and 74.7 parts of D-mannitol were loaded into a fluidized bed granulating apparatus, and sprayed with the binder liquid at a spray rate of 100 g/min to obtain granules. After the binder spraying, the resulting granules were dried to obtain a pharmaceutical composition (granulated product) of the present invention. Into a vessel-rotary mixer, 154.8 parts of the pharmaceutical composition (granulated product) of the present invention, 18 parts of microcrystalline cellulose, 5.4 parts of croscarmellose sodium, and 1.8 parts of magnesium stearate were loaded, and mixed. A rotary tabletting machine was used to obtain a pharmaceutical composition (tablets) of the present invention (a punch of 8 mm in diameter; a compression pressure of 8 kN/punch; a tablet weight of 180 mg).

### Example 4

A binder liquid was prepared by dissolving 10.8 parts of hydroxypropyl cellulose in 124.2 parts of purified water. Next, 149.4 parts of pulverized compound A maleate and 149.4 parts of D-mannitol were loaded into a fluidized bed granulating apparatus, and sprayed with the binder liquid at a spray rate of 100 g/min to obtain granules. After the binder spraying, the resulting granules were dried to obtain a pharmaceutical composition (granulated product) of the present invention. Into a vessel-rotary mixer, 309.6 parts of the pharmaceutical composition (granulated product) of the present invention, 36 parts of microcrystalline cellulose, 10.8 parts of croscarmellose sodium, and 3.6 parts of magnesium stearate were loaded, and mixed. A rotary tabletting machine was used to obtain a pharmaceutical composition (tablets) of the present invention (a punch of 10 mm in diameter; a compression pressure of 10 kN/punch; a tablet weight of 360 mg).

### Example 5

A binder liquid was prepared by dissolving 4.5 parts of hydroxypropyl cellulose in 51.75 parts of purified water. Next, 37.35 parts of pulverized compound A maleate and 35.55 parts of D-mannitol were loaded into a fluidized bed granulating apparatus (manufactured by Freund Corporation/Okawara MFG. Co., Ltd.; FLO-1), and sprayed with the binder liquid at a spray rate of 10 g/min to obtain granules. After the binder spraying, the resulting granules were dried to obtain a pharmaceutical composition (granulated product) of the present invention. Next, 77.4 parts of the pharmaceutical composition (granulated product) of the present invention, 9 parts of microcrystalline cellulose, 2.7 parts of croscarmellose sodium, and 0.9 parts of magnesium stearate were mixed using a mortar and pestle. A compression testing machine (manufactured by Shimadzu; Autograph; The same apparatus was used in the following Examples.) was used to obtain a pharmaceutical composition (tablets) of the present invention (a punch of 6 mm in diameter; a compression pressure of 6 kN/punch; a tablet weight of 90 mg).

### Example 6

To 77.4 parts of the pharmaceutical composition (granulated product) of the present invention obtained in Example 5, 2.4 parts of hydroxypropyl cellulose, 9 parts of microcrystalline cellulose, 2.7 parts of croscarmellose sodium, and 0.9 parts of magnesium stearate were added, and mixed using a mortar and pestle. A compression testing machine was used to obtain a pharmaceutical composition (tablets) of the present invention (a punch of 6 mm in diameter; a compression pressure of 6 kN/punch; a tablet weight of 92 mg).

### Example 7

To 77.4 parts of the pharmaceutical composition (granulated product) of the present invention obtained in Example 5, 5.0 parts of hydroxypropyl cellulose, 9 parts of microcrystalline cellulose, 2.7 parts of croscarmellose sodium, and 0.9 parts of magnesium stearate were added, and mixed using a mortar and pestle. A compression testing machine was used to obtain a pharmaceutical composition (tablets) of the present invention (a punch of 6 mm in diameter; a compression pressure of 6 kN/punch; a tablet weight of 95 mg).

### Example 8

A binder liquid was prepared by dissolving 2.7 parts of hydroxypropyl cellulose having a viscosity of 75 mPa·S (inclusive) to 150 mPa·S (inclusive) in a 5% aqueous solution at 25°C (manufactured by Hercules Inc.; product name: KLUCEL LF) in 31.05 parts of purified water. Next, 37.35 parts of pulverized compound A maleate and 37.35 parts of D-mannitol were loaded into a fluidized bed granulating apparatus (manufactured by Freund Co:cporation/Okawara MFG. Co., Ltd.; FLO-1), and sprayed with the binder liquid at a spray rate of 10 g/min to obtain granules. After the binder spraying, the resulting granules were dried to obtain a pharmaceutical composition (granulated product) of the present invention. Into a vessel-rotary mixer, 77.4 parts of the pharmaceutical composition (granulated product) of the present invention, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) of the present invention (a punch of 6 mm in diameter; a compression pressure of 6 kN/punch; a tablet weight of 90 mg).

### Example 9

A binder liquid was prepared by dissolving 2.7 parts of hydroxypropyl cellulose having a viscosity of 150 mPa·S (inclusive) to 400 mPa·S (inclusive) in a 5% aqueous solution at 25°C (manufactured by Hercules Inc.; product name: KLUCEL JF) in 31.05 parts of purified water. Next, 37.35 parts of pulverized compound A maleate and 37.35 parts of D-mannitol were loaded into a fluidized bed granulating apparatus (manufactured by Freund Corporation/Okawara MFG. Co., Ltd.; FLO-1), and sprayed with the binder liquid at a spray rate of 5 g/min to obtain granules. After the binder spraying, the resulting granules were dried to obtain a pharmaceutical composition (granulated product) of the present invention. Into a vessel-rotary mixer, 77.4 parts of the pharmaceutical composition (granulated product) of the present invention, 9 parts of microcrystalline cellulose, 2.7 parts of croscarmellose sodium, and 0.9 parts of magnesium stearate were loaded, and mixed. A rotary tabletting, machine was used to obtain a pharmaceutical composition (tablets) of the present invention (a punch of 6 mm in diameter; a compression pressure of 6 kN/punch; a tablet weight of 90 mg).

The formulations in Examples 1 to 7 are shown in Table 1.

**[Table 1]**

| Examples | 1 | 2 | 3 | 4 | 5 | 6 | 7 |
|---|---|---|---|---|---|---|---|
| Content of hydroxypropyl cellulose (%) | 3 | 3 | 3 | 3 | 5 | 7.5 | 10 |
| Percentage of hydroxypropyl cellulose to drug (%) | 22 | 7 | 7 | 7 | 12 | 18 | 25 |
| Compound A | 12.45 | 37.35 | 74.7 | 149.4 | 37.35 | 87.35 | 37.35 |
| D-mannitol | 62.25 | 37.35 | 74.7 | 149.4 | 35.55 | 35.55 | 35.55 |
| Hydroxypropyl cellulose | 2.7 | 2.7 | 5.4 | 10.8 | 4.5 | 6.9 | 9.5 |
| Microcrystalline cellulose | 9 | 9.0 | 18 | 36 | 9.0 | 9.0 | 9.0 |
| Croscarmellose sodium | 2.7 | 2.7 | 5.4 | 10.8 | 2.7 | 2.7 | 2.7 |
| Magnesium stearate | 0.9 | 0.9 | 1.8 | 3.6 | 0.9 | 0.9 | 0.9 |
| Total (mag) | 90.0 | 90.0 | 180.0 | 360.0 | 90.0 | 92.0 | 95.0 |

The formulations in Examples 8 to 9 are shown in Table 2.

**[Table 2]**

| Examples | 8 | 9 |
|---|---|---|
| Content of hydroxypropyl cellulose (%) | 3 | 3 |
| Percentage of hydroxypropyl cellulose to drug (%) | 7 | 7 |
| Compound A | 37.35 | 37.35 |
| D-mannitol | 37.35 | 37.35 |
| Hydroxypropyl cellulose | 2.7 | 2.7 |
| Microcrystalline cellulose | 9.0 | 9.0 |
| Croscarmellose sodium | 2.7 | 2.7 |
| Magnesium stearate | 0.9 | 0.9 |
| Total (mg) | 90.0 | 90.0 |

### Comparative Example 1

A binder liquid was prepared by dissolving 2.7 parts of polyvinylpyrrolidone K30 (manufactured by BASF; product name: Kollidon K30) in 31.05 parts of purified water. Next, 37.35 parts of pulverized compound A maleate and 37.35 parts of D-mannitol were loaded into a fluidized bed granulating apparatus (manufactured by Freund Corporation/Okawara MFG. Co., Ltd.; FLO-1), and sprayed with the binder liquid at a spray rate of 10 g/min to obtain granules. After the binder spraying, the resulting granules were dried to obtain a pharmaceutical composition (granulated product) for comparison. Into a vessel-rotary mixer, 77.4 parts of the pharmaceutical composition (granulated product) for comparison, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) for comparison (a punch of mm in diameter; a compression pressure of 6 kN/punch; a tablet weight of 90 mg).

### Comparative Examples 2

A binder liquid was prepared by dissolving 2.7 parts of hydroxypropyl cellulose having a viscosity of approximately 3.0 mPa·S (inclusive) to approximately 5.9 mPa ·S (inclusive) in a 2% aqueous solution at 20°C (manufactured by Nippon Soda Co., Ltd.; product name: HPC-SL; The same compound was used in the following Examples.) in 31.05 parts of purified water. Next, 37.35 parts of pulverized compound A maleate, and 37.35 parts of D-mannitol were loaded into a fluidized bed granulating apparatus (manufactured by Freund Corporation/Okawara MFG. Co., Ltd.; FLO-1), and sprayed with the binder liquid at a spray rate of 10 g/min to obtain granules. After the binder spraying, the resulting granules were dried to obtain a pharmaceutical composition (granulated product) for comparison. Into a vessel-rotary mixer, 77.4 parts of the pharmaceutical composition (granulated product) for comparison, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) for comparison (a punch of 6 mm in diameter; a compression pressure of 6 kN/punch; a tablet weight of 90 mg).

### Comparative Example 3

A binder liquid was prepared by dissolving 2.7 parts of hydroxypropyl methylcellulose 2910 (manufactured by Shin-Etsu Chemical Co., Ltd.; product name: TC-5R) in 31.05 parts of purified water. Next, 37.35 parts of pulverized compound A maleate and 37.35 parts of D-mannitol were loaded into a fluidized bed granulating apparatus (manufactured by Freund Corporation/Okawara MFG. Co., Ltd.; FLO-1), and sprayed with the binder liquid at a spray rate of 10 g/min to obtain granules. After the binder spraying, the resulting granules were dried to obtain a pharmaceutical composition (granulated product) for comparison. Into a vessel-rotary mixer, 77.4 parts of the pharmaceutical composition (granulated product) for comparison, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) for comparison (a punch of 6 mm in diameter; a compression pressure of 6 kN/punch; a tablet weight of 90 mg).

### Comparative Example 4

A binder liquid was prepared by dissolving 2.7 parts of hydroxypropyl cellulose (HPC-SL) in 31.05 parts of purified water. Next, 37.35 parts of pulverized compound A maleate and 37.35 parts of D-mannitol were loaded into a fluidized bed granulating apparatus, and sprayed with the binder liquid at a spray rate of 100 g/min to obtain granules. After the binder spraying, the resulting granules were dried to obtain a pharmaceutical composition (granulated product) for comparison. Into a vessel-rotary mixer, 77.4 parts of the pharmaceutical composition (granulated product) for comparison, 9 parts of microcrystalline cellulose, 2.7 parts of croscarmellose sodium, and 0.9 parts of magnesium stearate were loaded, and mixed. A rotary tabletting machine with a punch of which the surface had been mirror-polished was used to obtain a pharmaceutical composition (tablets) for comparison (a punch of 6 mm in diameter; a compression pressure of 6 kN/punch; a tablet weight of 90 mg).

### Comparative Example 5

Into a vessel-rotary mixer, 37.35 parts of pulverized compound A maleate, 40.05 parts of D-mannitol, 9 parts of microcrystalline cellulose, 2.7 parts of croscarmellose sodium, and 0.9 parts of magnesium stearate were loaded, and mixed. A rotary tabletting machine was used to obtain a pharmaceutical composition (tablets) for comparison (a punch of 6 mm in diameter; a compression pressure of 6 kN/punch; a tablet weight of 90 mg).

The formulations in Comparative Examples 1 to 5 are shown in Table 3.

**[Table 3]**

| Comparative Examples | 1 | 2 | 3 | 4 | 5 |
|---|---|---|---|---|---|
| Compound A | 37.35 | 37.35 | 37.35 | 37.35 | 37.35 |
| D-mannitol | 37.35 | 37.35 | 37.35 | 37.35 | 40.05 |
| Kollidon K30 | 2.7 | - | - | - | - |
| HPC-SL | - | 2.7 | - | 2.7 | - |
| TC-5R | - | - | 2.7 | - | - |
| Microcrystalline cellulose | 9.0 | 9.0 | 9.0 | 9.0 | 9.0 |
| Croscarmellose sodium | 2.7 | 2.7 | 2.7 | 2.7 | 2.7 |
| Magnesium stearate | 0.9 | 0.9 | 0.9 | 0.9 | 0.9 |
| Total (mg) | 90 | 90 | 90 | 90 | 90 |

### Experimental Example 1 [Evaluation of sticking to punches]

Whether or not the pharmaceutical compositions (tablets) of the present invention and the pharmaceutical compositions (tablets) for comparison sticked to a punch was evaluated. In this evaluation, a rotary tabletting machine (manufactured by Hata Iron Works Co., Ltd.; HT-X-SS-20; The same apparatus was used in the following Examples.) was used to form the granulated products prepared in Examples 1 to 4, 8, and 9 and Comparative Examples 1 to 5 into tablets under the conditions shown in Table 4 or 5, and the surface of each punch was visually observed, and further observed using a microscope (KEYENCE; Digital Microscope). When blurring of punches was observed, it was judged that the granulated product or the like sticked to the punch. By contrast, when blurring of punches was not observed, it was judged that the granulated product or the like did not stick to the punch. The results are shown in Tables 4 and 5.

**[Table 4]**

| Examples | 1 | 2 | 3 | 4 | 8 | 9 |
|---|---|---|---|---|---|---|
| (Number of tabletting)/ punch | 20000 tablets | 20000 tablets | 5000 tablets | 5000 tablets | 2000 tablets | 2000 tablets |
| Sticking to punches | Not observed | Not observed | Not observed | Not observed | Not observed | Not observed |

**[Table 5]**

| Comparative Examples | 1 | 2 | 3 | 4 | 5 |
|---|---|---|---|---|---|
| (Number of tabletting)/ punch | 250 tablets | 500 tablets | 300 tablets | 500 tablets | 100 tablets |
| Sticking to punches | Observed | Observed | Not observed | Observed | Observed |

### Experimental Example 2 [Evaluation of disintegrability]

The disintegrability and the tablet hardness of the pharmaceutical compositions (tablets) of the present invention and the pharmaceutical compositions (tablets) for comparison were evaluated. The disintegrability was evaluated in accordance with the general tests described in the Japanese Pharmacopoeia, using 6 tablets for each of tablets prepared in Examples 1 to 9 and Comparative Examples 1 to 5.
More particularly, water was used as a test fluid. A test basket, in which a tablet was placed, was attached to a vertical axis, and arranged in a beaker so that the test basket could be smoothly raised and lowered through a distance between 53 mm and 57 mm at a constant frequency between 29 and 32 cycles per minute. The test basket was adjusted so that the wire-mesh bottom face of the test basket was located 25 mm from the bottom of the beaker when the test basket was at the lowest point of the downward stroke. The volume of the test fluid in the beaker was such that the upper face of the test basket accorded with the surface of the test fluid when the test basket was at the lowest point of the downward stroke. The temperature of the test fluid was maintained at 37±2°C, and the time necessary for the disintegration of each tablet was measured. The average of values measured for 6 tablets was regarded as a disintegration time. As the tablet hardness, the average of values measured for 10 tablets is shown.

**[Table 6]**

| Examples | 1 | 2 | 3 | 4 | 5 | 6 | 7 | 8 | 9 |
|---|---|---|---|---|---|---|---|---|---|
| Disintegration time (Average) | 2 min 4 sec | 2 min 12 sec | 1 min 23 sec | 1 min 30 sec | 2 min 53 sec | 3 min 24 sec | 3 min 58 sec | 3 min 55 sec | 3 min 41 sec |
| Hardness (Average) | 86 N | 115 N | 140 N | 159 N | - | - | - | 118 N | 121 N |

**[Table 7]**

| Comparative Example | 1 | 2 | 3 | 4 | 5 |
|---|---|---|---|---|---|
| Disintegration time (Average) | 5 min 11 sec | 2 min 23 sec | 6 min 42 sec | 2 min 27 sec | 38 sec |
| Hardness (Average) | 127 N | 113 N | 107 N | 116 N | 96 N |

According to the pharmaceutical composition of the present invention, in which hydroxypropyl cellulose having a specific viscosity was used as a binder, the disintegrability of the composition was excellent (within 4 minutes), and failures in tabletting under compression-molding, such as sticking to punches, could be reduced, as apparent from Tables 4 to 7.

### INDUSTRIAL APPLICABILITY

The present invention relates to a pharmaceutical composition for oral administration containing 3-hydroxy-N'-(4-methoxybenzoyl)-N-[4-(4-methyl-1,4-diazepan-1-yl)benzoyl]-1,2-phenylenediamine or a pharmaceutically acceptable salt thereof and hydroxypropyl methylcellulose, and a use of hydroxypropyl methylcellulose in the manufacture of the pharmaceutical composition.
According to the present invention, failures in tabletting under compression-molding, such as sticking to punches, can be reduced, and disintegrability can be maintained.
As above, the present invention was explained with reference to particular embodiments, but modifications and improvements obvious to those killed in the art are included in the scope of the present invention.

## Claims

1. A pharmaceutical composition for oral administration, comprising 3-hydroxy-N¹-(4-methoxybenzoyl)-N²-[4 (4-methyl-1,4-diazepan-1-yl)benzoyl]-1,2-phenylenediamine or a pharmaceutically acceptable salt thereof, and hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C.

2. The pharmaceutical composition for oral administration according to claim 1, wherein the viscosity of hydroxypropyl cellulose is approximately 6 mPa·S (inclusive) to approximately 10 mPa·S (inclusive) in a 2% aqueous solution at 20°C.

3. The pharmaceutical composition for oral administration according to claim 1 or 2, wherein the amount of hydroxypropyl cellulose is approximately 2 w/w% to approximately 720 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof.

4. The pharmaceutical composition for oral administration according to any one of claims 1 to 3, wherein the amount of hydroxypropyl cellulose is approximately 4 w/w% to approximately 360 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof.

5. The pharmaceutical composition for oral administration according to any one of claims 1 to 4, wherein the amount of hydroxypropyl cellulose is approximately 7 w/w% to approximately 25 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof.

6. The pharmaceutical composition for oral administration according to any one of claims 1 to 5, **characterized in that** the composition is disintegrated within 5 minutes.

7. A process of manufacturing a pharmaceutical composition for oral administration, comprising mixing 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof with hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (inclusive) in a 5% aqueous solution at 25°C.

8. The process according to claim 7, wherein the viscosity of hydroxypropyl cellulose is approximately 6 mPa·S (inclusive) to approximately 10 mPa·S (inclusive) in a 2% aqueous solution at 20°C.

9. The process according to claim 7 or 8, wherein the amount of hydroxypropyl cellulose is approximately 2 w/w% to approximately 720 w/w% with respect to the amount of 3-hydroxy-N¹-(4-methoxybenzoyl)-N²-[4-(4-methyl-1,4-diazepan-1-yl)benzoyl]-l,2-phenylenediamine or a pharmaceutically acceptable salt thereof.

10. The process according to any one of claims 7 to 9, wherein the amount of hydroxypropyl cellulose is approximately 4 w/w% to approximately 360 w/w% with respect to the amount of 3-hydroxy-N¹-(4-methoxybenzoyl)-N²-[4-(4-methyl-1,4-diazepan-1-yl)benzoyl]-1,2-phenylenediamine or a pharmaceutically acceptable salt thereof.

11. The process according to any one of claims 7 to 10, wherein the amount of hydroxypropyl cellulose is approximately 7 w/w% to approximately 25 w/w% with respect to the amount of 3-hydroxy-N'-(4-methoxybenzoyl)-N'-[4-(4-methyl-1,4-diazepan-1-yl)benzoyl]-1,2-phenylenediamine or a pharmaceutically acceptable salt thereof.

12. The process according to any one of claims 7 to 11, **characterized in that** the composition is disintegrated within 5 minutes.

13. Use of hydroxypropyl cellulose having a viscosity of approximately 6 mPa·S (inclusive) to approximately 150 mPa·S (exclusive) in a 2% aqueous solution at 20°C, or a viscosity of 75 mPa·S (inclusive) to 400 mPa·S (incisive) in a 5% aqueous solution at 25°C in the manufacture of a pharmaceutical composition for oral administration, wherein disintearability is maintained and sticking to punches under compression-molding is prevented by adding the hydroxypropyl cellulose as a binder to 3-hydroxy-N'-(4-methoxybenzoyl)-N²-[4-(4-methyl-1,4-diazepan-1-yl)benzoyl] - 1,2-phenylenediamine or a pharmaceutically acceptable salt thereof.
